Europäisches Patentamt

European Patent Office

Office européen des brevets

(19)

(11) Veröffentlichungsnummer : **0 054 806**
**B1**

(12) **EUROPÄISCHE PATENTSCHRIFT**

(45) Veröffentlichungstag der Patentschrift :
20.02.85

(21) Anmeldenummer : 81110177.3

(22) Anmeldetag : 05.12.81

(51) Int. Cl.⁴ : **C 09 B   5/62**, C 07 D471/06

(54) **Verfahren zur Herstellung von Perylentetracarbonsäurediimid.**

(30) Priorität : **19.12.80 DE 3047991**

(43) Veröffentlichungstag der Anmeldung :
**30.06.82 Patentblatt 82/26**

(45) Bekanntmachung des Hinweises auf die Patenterteilung : **20.02.85 Patentblatt 85/08**

(84) Benannte Vertragsstaaten :
**CH DE GB LI**

(56) Entgegenhaltungen :
**DE-B- 1 138 876**
**FR-A- 1 580 971**
**US-A- 1 625 826**

(73) Patentinhaber : **BAYER AG
Konzernverwaltung RP Patentabteilung
D-5090 Leverkusen 1 Bayerwerk (DE)**

(72) Erfinder : **Schütze, Detlef-Ingo, Dr.
Altenberger Domstrasse 170
D-5060 Bergisch-Gladbach 2 (DE)**

Jouve, 18, rue St-Denis, 75001 Paris, France

## Beschreibung

Die Erfindung betrifft ein verbessertes Verfahren zur Herstellung von Perylentetracarbonsäurediimid durch Umsetzung von 1,8-Naphthalindicarbonsäureimid mit Alkali und Natriumacetat. Das entstehende Reaktionsprodukt wird, vorzugsweise direkt in der Reaktiongsschmelze zu Perylentetracarbonsäurediimid oxidiert.

Verfahren zur Herstellung von Perylentetracarbonsäurediimid sind seit Jahrzehnten bekannt. Bereits in der DE-PS 276 357, Bsp. 1 wird die Umsetzung von 1,8-Naphthalindicarbonsäureimid in Alkalihydroxid bei 290 °C-300 °C beschrieben. Das Reaktionsgemisch wird nach beendeter Kondensation auf Wasser gegeben und durch Einleiten von Luft oxidiert.

Eine Variante dieses Verfahrens ist in BIOS Final Report Nr. 1484, Seite 21 angegeben. 1,8-Naphthalindicarbonsäureimid wird in einer Mischung aus Kaliumhydroxid und Natriumacetat bei 220 °C umgesetzt und ebenfalls anschließend mit Luft in wäßrigem Medium oxidiert. Beide Verfahrensvarianten sind außerdem in der DE-OS 1 619 531 enthalten, wobei allerdings die Umsetzung des 1,8-Naphthalindicarbonsäureimids in Kaliumhydroxid und Natriumacetat bei 230-240 °C durchgeführt wird.

Das in der DE-PS 386 057 und in der DE-OS 1 619 531 beschriebene Verfahren zur Herstellung von Perylentetracarbonsäurediimid geht von Peryléntetracarbonsäure oder von ihrem Anhydrid aus, das mit Ammoniak kondensiert wird. Da jedoch Perylentetracarbonsäure aus Perylentetracarbonsäurediimid in konzentrierter Schwefelsäure bei Temperaturen oberhalb von 200 °C erhalten wird, stellt dieses Verfahren eine aufwendige und deshalb teure Verfahrensvariante dar.

Das neue Verfahren zur Herstellung von Perylentetracarbonsäurediimid durch Umsetzen von 1,8-Naphthalindicarbonsäureimid mit Alkali und Natriumacetat und Oxidieren des Reaktionsproduktes ist dadurch gekennzeichnet, daß die Umsetzung von 1,8-Naphthalindicarbonsäureimid in einer Mischung aus Kaliumhydroxid, Natriumhydroxid und Natriumacetat bei Temperaturen von 185 °C-210 °C, vorzugsweise 195 °C-200 °C vorgenommen wird, und man das Reaktionsprodukt, vorzugsweise in der Reaktionsschmelze, oxidiert.

Die Oxidation kann in üblicher Weise durch Austragen des Reaktionsansatzes auf Wasser und Einleiten von Luft oder durch Umsetzung mit üblichen Oxidationsmitteln erfolgen. Besonders vorteilhaft oxidiert man jedoch direkt in der Reaktionsschmelze. Diese Verfahrensvariante weicht ab von allen bisher bekannten Verfahren, da sie die Oxidation nach Austragen der Schmelze auf Wasser vorsehen.

Für 1 Teil 1,8-Naphthalindicarbonsäureimid werden vorzugsweise 1-2 Teile, besonders bevorzugt 1,1-1,5 Teile, Natriumhydroxid, 3-5 Teile, besonders bevorzugt 3,5-4,5 Teile, Kaliumhydroxid und 1-1,5 Teile, besonders bevorzugt 1,1-1,3 Teile, Natriumacetat eingesetzt.

Die Oxidation kann beispielsweise mit Sauerstoff, Sauerstoff enthaltenden Gasen, z. B. Luft, oder durch Zugabe von oxidierend wirkenden Salzen wie beispielsweise Nitraten, Dichromaten, Chloraten, Peroxodisulfaten, Addukten von Wasserstoffperoxid an Borate oder Carbonate u. a. durchgeführt werden. Bevorzugt ist Luft.

Das neue Verfahren liefert auf einfache Weise Perylentetracarbonsäurediimid von hoher Qualität und in guten Ausbeuten.

Ein weiterer Vorteil des neuen Verfahrens bei Oxidation in der Schmelze besteht darin, daß die Reaktionszeit nach Eintragen des 1,8-Naphthalindicarbonsäureimids bis zum Austragen auf Wasser und Isolieren ca. 3 Stunden beträgt, während beispielsweise nach der Vorschrift BIOS Final Report Nr. 1484, Seite 21, für Kondensation und Oxidation 23 Stunden benötigt werden.

Daß diese drastische Herabsetzung der Reaktionszeit nicht Ausbeute und Qualität des Reaktionsproduktes beeinträchtigt, muß als überraschend angesehen werden.

Außerdem ergeben sich für die Durchführung weitere Vorteile dadurch, daß beim Eintragen des 1,8-Naphthalindicarbonsäureimids in die Schmelze kein Schäumen auftritt, wie es nach den bekannten Verfahren bei höheren Temperaturen der Fall ist. Deshalb ist es beispielsweise möglich, bereits bei 185 °C zügig alles 1,8-Naphthalindicarbonsäureimid einzutragen, und man erhält somit eine weitere Verkürzung der Reaktionszeit.

Die Aufarbeitung der Reaktionsschmelze nach der Oxidation erfolgt durch Zugabe von Wasser und Isolieren eines feuchten Preßkuchens, der anschließend in verdünnter Säure, z. B. Salzsäure, ausgekocht wird. Nach erneutem Isolieren wird getrocknet. Erfolgte die Oxidation im wäßrigen Milieu, wird das Reaktionsprodukt in üblicher Weise abgetrennt und gereinigt.

Das erhaltene Perylentetracarbonsäurediimid zeichnet sich durch eine hohe Qualität aus und kann entweder durch geeignete Verfahren direkt in ein Pigment überführt werden oder beispielsweise als Ausgangsmaterial für die Herstellung von Perylentetracarbonsäure, die wiederum ein Ausgangsmaterial für Pigmente oder Küpenfarbstoffe darstellt, oder für Pigment Rot 179 (N,N'-Dimethyl-perylentetracarbonsäurediimid) dienen.

Beispiel 1

584 g KOH-Pulver und 175 g NaOH-Pulver werden gemischt und auf 180 °C erhitzt. Anschließend werden 188 g wasserfreies Natriumacetat zugesetzt, auf 185 °C geheizt und zügig 150 g 1,8-Naphthalindicarbonsäureimid eingetragen. Danach wird auf 195 °C erwärmt und unter gleichzeitigem Überleiten von 20 Litern Luft pro Stunde 2 1/2 Stunden bei dieser Temperatur gerührt. Nach beendeter Reaktion werden 750 ml Wasser zugetropft und danach die Schmelze in 5 Liter Wasser eingerührt. Nach kurzzeitigem Erhitzen zum Sieden wird abgesaugt und mit Wasser gewaschen. Der feuchte Preßkuchen wird in 2 Litern Wasser, das mit 100 ml 36 %iger Salzsäure angesäuert worden ist, zum Sieden erhitzt, erneut abgesaugt, neutral gewaschen und getrocknet. Man erhält 141 g Perylentetracarbonsäurediimid mit einem Reingehalt von 98,2 %. Das entspricht einer Reinausbeute von 93,4 % d. Th.

Beispiel 2

531 g KOH-Pulver und 219 g NaOH-Pulver werden gemischt und auf 180 °C erhitzt. Bei dieser Temperatur werden dann unter Stickstoff 171 g wasserfreies Natriumacetat zugesetzt und anschließend bei 185 °C zügig 150 g 1,8-Naphthalindicarbonsäureimid eingetragen. Die Schmelze wird auf 200 °C geheizt und 1 Stunde bei dieser Temperatur gerührt. Danach wird innerhalb von 1 Stunde portionsweise mit einer Mischung bestehend aus 20,6 g Natriumnitrat und 13,7 g Kaliumnitrat oxidiert. Es wird noch 1 Stunde bei 200 °C nachgerührt und anschließend wie in Beispiel 1 zur Isolierung des Perylentetracarbonsäurediimids verfahren.

Man erhält 138 g Perylentetracarbonsäurediimid mit einem Reingehalt von 96,1 % d. Th. Das entspricht einer Reinausbeute von 89,4 % d. Th.

Beispiel 3

480 g KOH-Pulver und 220 g NaOH-Pulver werden gemischt und auf 180 °C erhitzt. Dann werden 170 g wasserfreies Natriumacetat unter Stickstoff eingetragen und die Temperatur auf 185 °C gesteigert. Bei dieser Temperatur werden 150 g 1,8-Naphthalindicarbonsäureimid zügig eingetragen. Die Schmelze wird auf 195 °C geheizt und 1/2 Stunde bei dieser Temperatur gerührt. Anschließend wird innerhalb von 1 Stunde durch Zugabe von 103 g Kaliumperoxodisulfat oxidiert. Es wird 1 Stunde nachgerührt und danach wie in Beispiel 1 zur Isolierung des Perylentetracarbonsäurediimids verfahren.

Man erhält 139 g Perylentetracarbonsäurediimid mit einem Reingehalt von 95,4 % d. Th. Das entspricht einer Reinausbeute von 89,3 % d. Th.

Ansprüche

1. Verfahren zur Herstellung von Perylentetracarbonsäurediimid durch Umsetzen von 1,8-Naphthalindicarbonsäureimid mit Alkali und Natriumacetat und Oxidieren des Reaktionsprodukts, dadurch gekennzeichnet, daß die Umsetzung von 1,8-Naphthalindicarbonsäureimid in einer Mischung aus Natriumhydroxid, Kaliumhydroxid und Natriumacetat bei Temperaturen von 185-210 °C vorgenommen wird, und das Reaktionsprodukt, vorzugsweise in der Reaktionsschmelze, oxidiert.

2. Verfahren gemäß Anspruch 1, dadurch gekennzeichnet, daß man pro Teil 1,8-Naphthalindicarbonsäureimid 1-2 Teile Natriumhydroxid, 3-5 Teile Kaliumhydroxid und 1-1,5 Teile Natriumacetat einsetzt.

3. Verfahren gemäß Anspruch 1, dadurch gekennzeichnet, daß man pro Teil 1,8-Naphthalindicarbonsäureimid 1,1-1,5 Teile Natriumhydroxid, 3,5-4,5 Teile Kaliumhydroxid und 1,1-1,3 Teile Natriumacetat einsetzt.

4. Verfahren gemäß den Ansprüchen 1-3, dadurch gekennzeichnet, daß man als Oxidationsmittel Sauerstoff, Sauerstoff enthaltende Gase, oxidierend wirkende Salze oder Addukte von Wasserstoffperoxid an Borate oder Carbonate verwendet.

Claims

1. Process for the preparation of perylenetetracarboxylic acid diimide by reacting 1,8-naphthalenedicarboxylic acid imide with alkali and sodium acetate and oxidising the reaction product, characterised in that the reaction of 1,8-naphthalenedicarboxylic acid imide is carried out in a mixture of sodium hydroxide, potassium hydroxide and sodium acetate at temperatures of 185-210 °C and the reaction product is oxidised preferably in the reaction melt.

2. Process according to Claim 1, characterised in that per part of 1,8-naphthalenedicarboxylic acid imide 1-2 parts of sodium hydroxide, 3-5 parts of potassium hydroxide and 1-1.5 parts of sodium acetate are used.

3. Process according to Claim 1, characterised in that per part of 1,8-naphthalenedicarboxylic acid imide 1.1-1.5 parts of sodium hydroxide, 3.5-4.5 parts of potassium hydroxide and 1.1-1.3 parts of sodium acetate are used.

4. Process according to Claims 1-3, characterised in that oxygen, oxygen-containing gases, salts which have an oxidising action or adducts of hydrogen peroxide to borates or carbonates are used as the oxidising agent.

Revendications

1. Procédé pour la fabrication de diimide d'acide pérylènetétracarboxylique par réaction de l'imide d'acide 1,8-naphtalènedicarboxylique avec un alcali et l'acétate de sodium et oxydation du produit de réaction, caractérisé en ce que la réaction de l'imide d'acide 1,8-naphtalènedicarboxylique est effectuée dans un mélange d'hydroxyde de sodium, d'hydroxyde de potassium et d'acétate de sodium à des températures de 185-210 °C, et on oxyde le produit de réaction, de préférence dans la masse réactionnelle fondue.

2. Procédé selon la revendication 1, caractérisé en ce que l'on utilise 1-2 parties d'hydroxyde de sodium, 3-5 parties d'hydroxyde de potassium et 1-1,5 partie d'acétate de sodium par partie d'imide d'acide 1,8-naphtalènedicarboxylique.

3. Procédé selon la revendication 1, caractérisé en ce que l'on utilise 1,1-1,5 partie d'hydroxyde de sodium, 3,5-4,5 parties d'hydroxyde de potassium et 1,1-1,3 partie d'acétate de sodium par partie d'imide d'acide 1,8-naphtalènedicarboxylique.

4. Procédé selon les revendications 1-3, caractérisé en ce que l'on utilise comme agents d'oxydation l'oxygène, des gaz contenant de l'oxygène, des sels à action oxydante ou des produits d'addition de peroxyde d'hydrogène sur des borates ou des carbonates.